# EUROPÄISCHE PATENTSCHRIFT

(11) **EP 0 597 278 B1**
(45) Veröffentlichungstag und Bekanntmachung des Hinweises auf die Patenterteilung: **03.09.1997**
(21) Anmeldenummer: 93116771.2
(22) Anmeldetag: 18.10.1993
(51) Int. Cl.: C10L 1/22

(54) **Erdölmitteldestillatzusammensetzungen**
Mineral-oil middledistillate compositions
Compositions de distillats moyens de pétrole

(30) Priorität: 07.11.1992 DE 4237662
(43) Veröffentlichungstag der Anmeldung: 18.05.1994
(73) Patentinhaber: BASF Aktiengesellschaft, 67063 Ludwigshafen (DE)
(72) Erfinder: Dralle-Voss, Gabriele, Dr., D-64297 Darmstadt (DE); Oppenlaender, Knut, Dr., D-67061 Ludwigshafen (DE); Barthold, Klaus, Dr., D-68259 Mannheim (DE); Wenderoth, Bernd, Dr., D-68623 Lampertheim (DE); Kasel, Wolfgang, Dr., D-69226 Nussloch (DE)

(56) Entgegenhaltungen:
- EP-A- 0 301 448
- EP-A- 0 398 101
- US-A- 3 173 770
- US-A- 3 202 491

## Beschreibung

Die Erfindung betrifft Erdölmitteldestillatzusammensetzungen auf der Basis eines Kohlenwasserstoffgemisches, die als Paraffindispergator Umsetzungsprodukte von Aminoalkylencarbonsäuren mit primären oder sekundären langkettigen Aminen enthalten.

Mitteldestillate wie z.B. Gasöle, Dieselöle oder Heizöle, die durch Destillation aus Erdölen gewonnen werden, haben je nach Herkunft des Rohöls unterschiedliche Gehalte an Paraffinen. Bei tieferen Temperaturen kommt es zur Ausscheidung fester Paraffine (Trübungspunkt oder Cloud Point, CP). Bei weiterer Abkühlung bilden die plättchenförmigen n-Paraffinkristalle eine "Kartenhausstruktur" und das Mitteldestillat stockt, obwohl der überwiegende Teil des Mitteldestillates noch flüssig ist. Durch die ausgefallenen n-Paraffine im Temperaturgebiet zwischen Trübungspunkt und Stockpunkt (bzw. Pour Point) wird die Fließfähigkeit der Erdölmitteldestillate erheblich beeinträchtigt. Die Paraffine verstopfen Filter und verursachen ungleichmäßige oder völlig unterbrochene Kraftstoffzufuhr zu den Verbrennungsaggregaten.

Es ist seit langem bekannt, daß durch geeignete Zusätze das Kristallwachstum der Paraffine in den Erdölmitteldestillate modifiziert werden kann. Gut wirksame Additive verhindern einerseits, daß Mitteldestillate derartige Kartenhaus-Strukturen ausbilden und bei Temperaturen wenige Grad Celsius unterhalb der Temperatur, bei welcher die ersten Paraffinkristalle auskristallisieren, bereits fest werden und andererseits feine, gut kristallisierte, separate Paraffinkristalle bilden, welche Filter in Kraftfahrzeugen und Heizungsanlagen passieren oder zumindest einen für den flüssigen Teil der Mitteldestillate durchlässigen Filterkuchen bilden, so daß ein störungsfreier Betrieb sichergestellt ist.

Ein Nachteil dieses Standes der Technik beruht darauf, daß die ausgefallenen Paraffinkristalle aufgrund ihrer gegenüber dem flüssigen Teil höheren Dichte dazu neigen, sich beim Lagern mehr und mehr am Boden des Behälters abzusetzen. Dadurch bildet sich eine im oberen Behälterteil homogene paraffinarme Phase und am Boden eine zweiphasige paraffinreiche Schicht. Da sowohl in Fahrzeugtanks als auch in Lager- oder Liefertanks der Mineralölhändler der Abzug des Mitteldestallates meist wenig oberhalb des Behälterbodens erfolgt, besteht die Gefahr, daß die hohe Konzentration an festen Paraffinen zu Verstopfungen von Filtern und Dosiereinrichtungen führt. Diese Gefahr wird umso größer, je weiter die Lagertemperatur die Ausscheidungstemperatur der Paraffine (Trübungspunkt) unterschreitet, da die ausgeschiedene Paraffinmenge eine Funktion der Temperatur darstellt und mit sinkender Temperatur ansteigt.

Bei den Paraffinkristallmodifikatoren, den sog. Fließverbesserern, handelt es sich um Polymere, die durch Co-Kristallisation (Interaktion) das Kristallwachstum der n-Paraffine verändern. Dabei werden die Fließeigenschaften des Mitteldestillats bei niedrigeren Temperaturen positiv beeinflußt. Die Wirksamkeit der Fließverbesserer wird nach DIN 51428 indirekt durch Messung des "Cold Filters Plugging Points" ("CFPP") ausgedrückt.

Als Kältefließverbesserer werden an sich bekannte Ethylencopolymerisate, vor allem Copolymere von Ethylen und ungesättigten Estern, verwendet. DE 11 47 799 und DE 19 14 756 beschreiben beispielsweise Copolymerisate des Ethylens mit Vinylacetat, mit einem Gehalt von 25 bis 45 Gew.-% Vinylacetat oder Vinylpropionat und einem Molekulargewicht von 500 bis 5000.

Weiterhin ist aus GB 2 095 698 bekannt, Mitteldestillaten eine Kombination aus den genannten Copolymeren mit Amiden aus langkettigen Aminen und aromatischen oder cycloaliphatischen Carbonsäuren zuzusetzen.

Bezüglich der Dispergiereigenschaften der ausgeschiedenen Paraffine befriedigen diese Mischungen aber noch nicht.

Es ist daher erforderlich, daß die zugesetzten Additive auch die Dispergierung der ausgeschiedenen Paraffine bewirken.

Aus der EP 398 101-A sind Umsetzungsprodukte von Aminoalkylenpolycarbonsäuren mit langkettigen sekundären Aminen, die vollständig mit dem Amin zum Amid oder Ammoniumsalz umgesetzt worden sind, als Paraffindispergatoren bekannt. Um eine gute Dispergierung der ausgeschiedenen Paraffine mit diesen Produkten zu erhalten, muß dem Dieselkraftstoff neben dem Dispergator jedoch oft noch 0,25 bis 40 ppm eines Leitfähigkeitsverbesserers zugesetzt werden.

Die Zumischung eines weiteren Additivs ist jedoch produktionstechnisch nachteilig und kostenintensiv. Außerdem kann die Zumischung eines Leitfähigkeitsverbesserers gegebenenfalls ökologisch nachteilig sein.

Es bestand daher die Aufgabe, Paraffindispergatoren für Erdölmitteldestillate zu finden, die auch ohne zusätzlichen Leitfähigkeitsverbesserer eine ausgezeichnete Dispergierwirkung aufweisen.

Diese Aufgabe wird gelöst durch Erdölmitteldestillatzusammensetzungen auf der Basis eines Kohlenwasserstoffgemisches, dessen Siedebeginn über 160°C und dessen Siedeende unter 420°C liegt, enthaltend eine als Paraffindispergator wirksame Menge der Verbindungen der Formeln I und/oder II 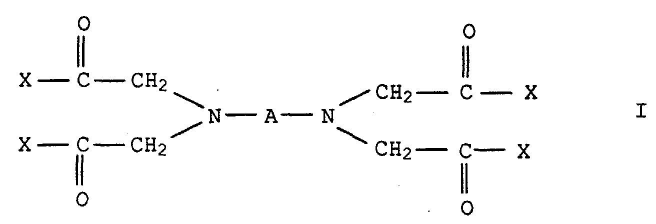 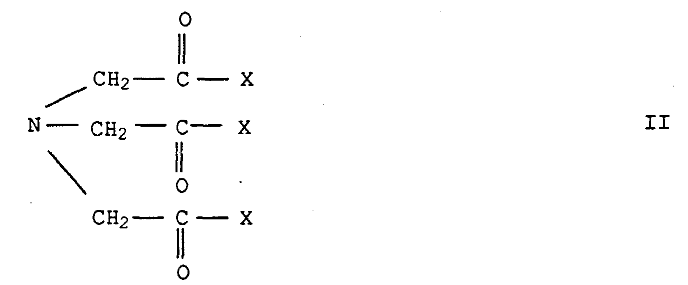 worin bedeuten
- A: einen geradkettigen oder verzweigten Alkylenrest mit 2 bis 6 Kohlenstoffatomen oder den Rest der Formel III 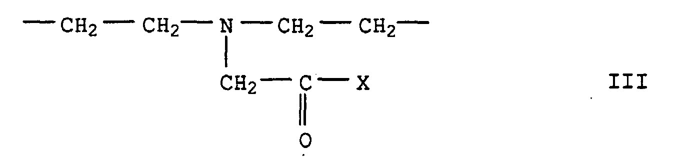
- X: die Reste

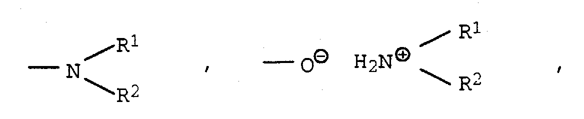

―OH und/oder ―o^{⊖} M^{⊕}

wobei R¹ und R² ein geradkettiger aliphatischer Rest mit 10 bis 30 Kohlenstoffatomen sind und M ein Alkali- oder Erdalkalimetall ist,
mit der Maßgabe, daß die folgenden beiden Bedingungen erfüllt sind, wonach X
a) mindestens eine 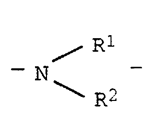 Gruppe und
b) mindestens eine -OH- und/oder
   - O^{⊖} M^{⊕} -Gruppe bedeutet.

Überraschenderweise wurde gefunden, daß bei Verwendung der Verbindungen der Formeln I und/oder II, d.h. der Umsetzungsprodukte von Aminoalkylencarbonsäuren (III und IV) 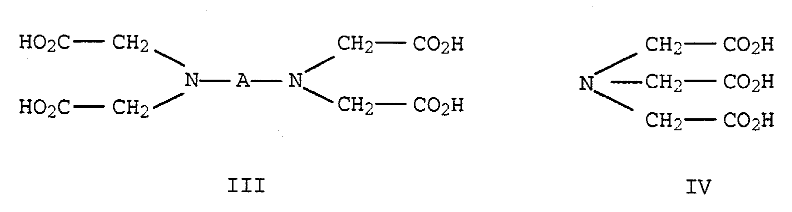 worin A die oben angegebene Bedeutung hat, mit sekundären langkettigen Aminen mit mindestens einer Amid- und einer Carbonsäure und/oder Carboxylat-Gruppe, auf den Zusatz eines Leitfähigkeitsverbesserers verzichtet werden kann, um den gleichen Effekt der Dispergierung der ausgeschiedenen Paraffine zu erhalten.

Die erfindungsgemäßen Erdölmitteldestillatzusammensetzungen weisen somit eine verbesserte Kältefließfähigkeit durch besserere Dispergierung der ausgeschiedenen Paraffinkristalle auf.

Die in den Erdölmitteldestillaten eingesetzten Verbindungen der Formeln I und/oder II weisen bevorzugt Reste R¹ und R² auf, bei denen R¹ und R² einen geradkettigen aliphatischen Rest mit 14 bis 22 Kohlenstoffatomen bedeuten. Bei den zur Herstellung der Verbindungen eingesetzten Aminen handelt es sich um sekundäre Amine. Als sekundäre Amine seien im einzelnen Dioleylamin, Ditalgfettamin, Dipalmitinamin, Dikokosfettamin und Dibehenylamin und vorzugsweise Distearylamin oder hydriertes Ditalgfettamin (letztere jeweils mit 16 bis 18 C-Atomen) genannt.

Der Umsetzungsgrad der Aminoalkylenpolycarbonsäure (III, IV) mit dem Amin liegt zweckmäßigerweise zwischen 1 : 1,5 - 1 : 3.

Die nicht umgesetzten Carboxylgruppen können als freie Carbonsäuren vorliegen oder sie können als Carboxylate vorliegen, die entweder durch innermolekulare Neutralisation mit den Aminoalkylengruppen (Betainbildung) oder durch Neutralisation mit Metallsalzen gebildet werden: 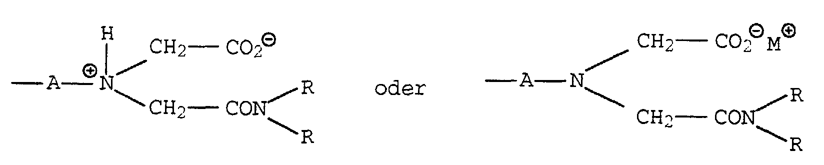 Besonders bevorzugt sind Verbindungen der folgenden Formel V: 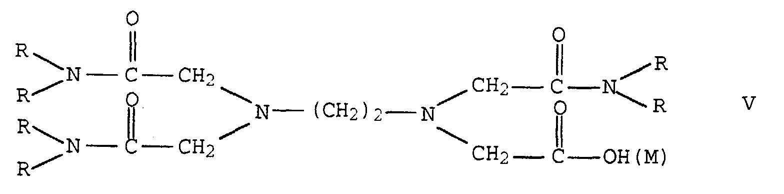 d.h. X bedeutet dreimal den Rest 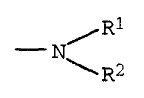 und einmal den Rest ―OH oder ―O^{⊖} M^{⊕}.

Die Verbindungen der Formeln I bzw. II werden den erfindungsgemäßen Erdölmitteldestillatzusammensetzungen in der Regel in Mengen von 25 bis 1000 ppm, bevorzugt 50 bis 500 ppm, zugesetzt.

Üblicherweise enthalten die Mitteldestillate bereits an sich bekannte Fließverbesserer, die in der Patentliteratur eingehend beschrieben sind, z.B. Ethylen-Vinylester-Copolymerisate und deren Mischungen mit anderen Copolymeren (gemäß DE 19 14 756 und DE 40 36 227), Alkylfumarat-Vinylacetat-Copolymere (gemäß EP 155 807) oder α-olefin-Maleinsäureanhydridester.

Es kommen jedoch auch gleichermaßen Terpolymere in Betracht, die neben Ethylen und Vinylestern oder Acrylestern noch weitere Comonomere enthalten. Das Molekulargewicht dieser Fließverbesserer beträgt in der Regel 500 bis 5000, vorzugsweise 1000 bis 3000.

Auch Mischungen verschiedener Fließverbesserer kommen in Betracht.

### Beispiele

### A) Synthese der Aminoalkylenpolycarbonsäureamide

PD1: Herstellung des Ethylendiamintetraessigsäuredistearylfettamintrisamids:
1010,2 g (2 Mol) PD Distearylfettamin (hydriertes Ditalgfettamin) werden aufgeschmolzen und mit 6 g p-Toluolsulfonsäure und 194,6 g (0,67 Mol) Ethylendiamintetraessigsäure versetzt.

Das Reaktionsgemisch wird auf 190°C erhitzt und 3 h bei dieser Temperatur kondensiert. Das Produkt wird anschließend heiß filtriert. Man erhält 1180 g eines braunen Feststoffs mit einer Säurezahl von 33 mg KOH/g.

PD2: Herstellung des Calciumsalzes von PD1
101 g (0,2 Mol) PD1) werden aufgeschmolzen und mit 14,8 g Calciumhydroxid versetzt. Es wird 2 h bei 100°C erhitzt, das Produkt wird anschließend filtriert. Man erhält ca. 110 g eines braunen Feststoffs.

### B) Prüfung der Erdölmitteldestillatzusammensetzungen

Beschreibung der Testmethode:
Die Mitteldestillate wurden mit unterschiedlichen Mengen an Paraffindispergatoren PD 1, PD 2 bzw. PD 3 und/oder Fließverbesserer F1, gegebenenfalls zusätzlich mit Leitfähigkeitsverbesserer LV (E) (Vergleich), bei 40°C unter Rühren additiviert und anschließend auf Raumtemperatur abgekühlt.

Die additivierten Mitteldestillate wurden in 100-ml-Meßzylindern für 20 Stunden in einem Kälteschrank bei -13°C gelagert. Anschließend wurde visuell das Volumen der sedimentierten Paraffinphase (Vol-%) und das Aussehen der Ölphase beurteilt.

Folgende Erdölmitteldestillatzusammensetzungen wurden geprüft:
Erdölmitteldestillatzusammensetzungen, enthaltend
1) als Paraffindispergator
   PD 1, PD 2 Ethylendiamintetraessigsäure-Derivate gemäß A),
2) als Fließverbesserer
   Fl 1 Ethylen/Vinylpropionat (mit ca. 40 Gew.-% Vinylpropionat) mit einem mittleren Molekulargewicht von ca. 2500

### C) Erdölmitteldestillatzusammensetzungen (Vergleich analog EP 398 101), enthaltend

1) als Paraffindispergator
   PD 3 Nitriloessigsäureamid A)1 aus EP 398 101
2) als Fließverbesserer
   Fl 1 Ethylen/Vinylpropionat (mit ca. 40 Gew.-% Vinylpropionat) mit einem mittleren Molekulargewicht von ca. 2500
3) als Leitfähigkeitsverbesserer
   LV (E) aus EP 398 101

Als Mitteldestillate wurden für die folgenden Dispergierversuche Dieselkraftstoffe in handelsüblicher deutscher Raffineriequalität verwendet; sie werden als DK 1, DK 2, DK 3 bezeichnet:

| | DK 1 | DK 2 | DK 3 |
|---|---|---|---|
| Trübungspunkt CP (°C) | -8 | -8 | -7 |
| CFPP (°C) | -13 | -12 | -10 |
| Dichte b. 20°C (g/ml) | 0,827 | 0,831 | 0,829 |
| Siedeanfang (°C) | 165 | 175 | 183 |
| 20 % Siedepunkt (°C) | 210 | 223 | 211 |
| 90 % Siedepunkt (°C) | 318 | 314 | 317 |
| Siedeende (°C) | 358 | 352 | 364 |

In den Tabellen 1 bis 3 sind die Ergebnisse aufgeführt. Es wird ersichtlich, daß die Verbindungen PD 1 und PD 2 eine bessere Dispergierwirkung zeigen als die aus der EP 398 101 bekannte Verbindung, die nur in Gegenwart eines Leitfähigkeitsverbesserers vergleichbare Dispergiereffekte aufweist.

**Tabelle 1**

| DK 1, CP: -8°C, CFPP: -13°C | | | | | | |
|---|---|---|---|---|---|---|
| PD 1 (ppm) | PD 2 (ppm) | PD 3 (ppm) | FI 1 (ppm) | LV (E) (ppm) | Paraffin-Sediment (Vol.%) | Aussehen Ölphase |
| 100 | - | - | 200 | - | 0 | dispergiert |
| - | - | 100 | 200 | 1 | 35 | dispergiert |

**Tabelle 2**

| DK 2, CP: -8°C, CFPP: -12°C | | | | | | |
|---|---|---|---|---|---|---|
| PD 1 (ppm) | PD 2 (ppm) | PD 3 (ppm) | FI 1 (ppm) | LV (E) (ppm) | Paraffin-Sediment (Vol.%) | Aussehen Ölphase |
| 100 | - | - | 300 | - | 15 | dispergiert |
| - | 100 | - | 300 | - | 20 | dispergiert |
| - | - | 100 | 300 | 1 | 30 | dispergiert |
| - | - | 100 | 300 | - | 25 | trüb |
| - | - | - | 300 | - | 35 | klar |

**Tabelle 3**

| DK 3, CP: -7°C, CFPP: -10°C | | | | | | |
|---|---|---|---|---|---|---|
| PD 1 (ppm) | PD 2 (ppm) | PD 3 (ppm) | FI 1 (ppm) | LV (E) (ppm) | Paraffin-Sediment (Vol.%) | Aussehen Ölphase |
| 100 | - | - | 300 | - | 20 | dispergiert |
| - | 100 | - | 300 | - | 10 | dispergiert |
| - | - | 100 | 300 | 1 | 15 | dispergiert |
| - | - | 100 | 300 | - | 10 | trüb |
| - | - | - | 300 | - | 10 | klar |

## Patentansprüche

1. Erdölmitteldestillatzusammensetzungen auf der Basis eines Kohlenwasserstoffgemisches, dessen Siedebeginn über 160°C und dessen Siedeende unter 420°C liegt, enthaltend eine als Paraffindispergator wirksame Menge der Verbindungen der Formeln I und/oder II 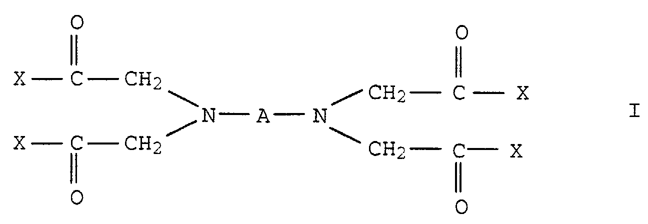 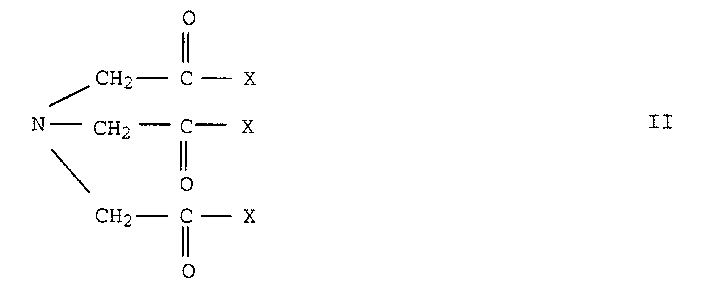 worin bedeuten
A einen geradkettigen oder verzweigten Alkylenrest mit 2 bis 6 Kohlenstoffatomen oder den Rest der Formel III 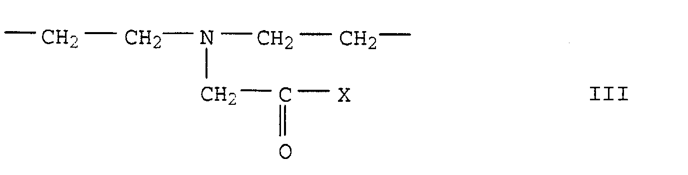
X die Reste 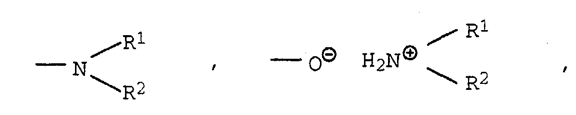
- OH und/oder ―O^{⊖} M^{⊕}
wobei R¹ und R² ein geradkettiger aliphatischer Rest mit 10 bis 30 Kohlenstoffatomen sind und M ein Alkali- oder Erdalkalimetall ist,
mit der Maßgabe, daß die folgenden beiden Bedingungen erfüllt sind, wonach X
a) mindestens eine 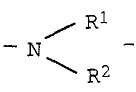 Gruppe und
b) mindestens eine -OH- und/oder
-O^{⊖} M^{⊕} -Gruppe bedeutet.

2. Erdölmitteldestillatzusammensetzungen nach Anspruch 1, dadurch gekennzeichnet, daß sie Verbindungen der Formeln I und/oder II enthalten, worin R¹ und R² einen geradkettigen aliphatischen Rest mit 14 bis 22 Kohlenstoffatomen bedeuten.

3. Erdölmitteldestillatzusammensetzungen nach Anspruch 1, dadurch gekennzeichnet, daß sie Verbindungen der Formel I enthalten,
in der X dreimal für den Rest 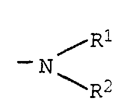 und einmal für den Rest -OH oder -O^{⊖} M^{⊕} steht.

4. Erdölmitteldestillatzusammensetzungen nach Anspruch 2, dadurch gekennzeichnet, daß R¹ und R² einen geradkettigen aliphatischen Rest mit 16 bis 18 Kohlenstoffatomen bedeuten.

## Claims

1. A mineral oil middle distillate composition based on a hydrocarbon mixture whose boiling range begins at above 160°C and ends at below 420°C, containing an amount, effective as a paraffin dispersant, of a compound of the formulae I and/or II 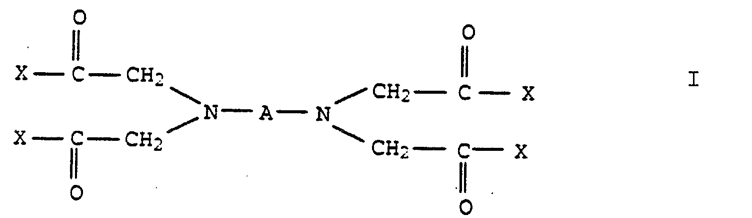 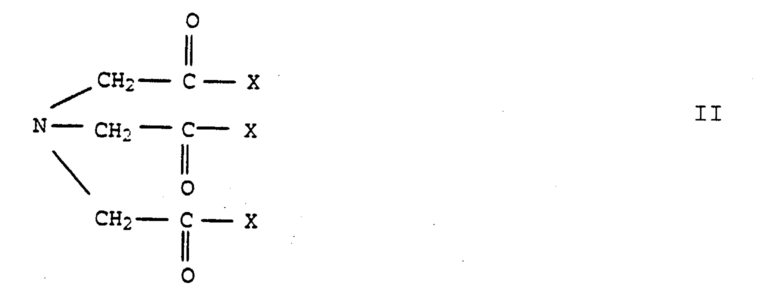 where
A is straight-chain or branched alkylene of 2 to 6 carbon atoms or a radical of the formula III 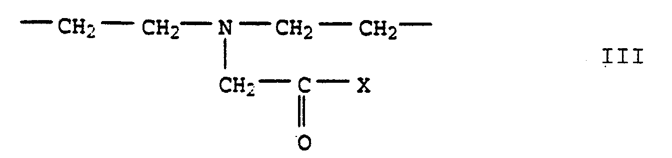
X is 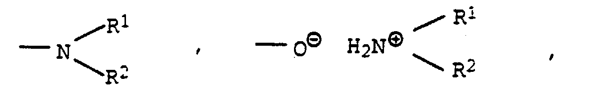
-OH oder -O^{⊖} M^{⊕}
R¹ and R² are each a straight-chain aliphatic radical of 10 to 30 carbon atoms and
M is an alkali metal or alkaline earth metal,
with the proviso that the following two conditions are fulfilled, ie. X is
a) at least one 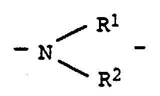 group and
b) at least one -OH- and/or -O^{⊖}M^{⊕} group.

2. A mineral oil middle distillate composition as claimed in claim 1, which contains a compound of the formulae I and/or II, where R¹ and R² are each a straight-chain aliphatic radical of 14 to 22 carbon atoms.

3. A mineral oil middle distillate composition as claimed in claim 1, which contains a compound of the formula I where three radicals X are each 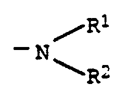 and one radical X is -OH or -O^{⊖}M^{⊕}.

4. A mineral oil middle distillate composition as claimed in claim 2, wherein R¹ and R² are each a straight-chain aliphatic radical of 16 to 18 carbon atoms.

## Revendications

1. Compositions de distillats moyens de pétrole à base d'un mélange d'hydrocarbures, dont le début d'ébullition est supérieur à 160°C et la fin d'ébullition est inférieure à 420°C, contenant une quantité suffisante, pour avoir une dispersion de la paraffine, des composés de formules I et/ou II 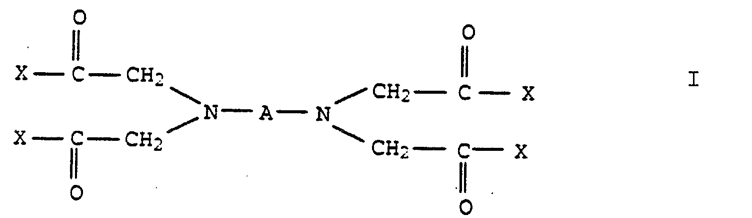 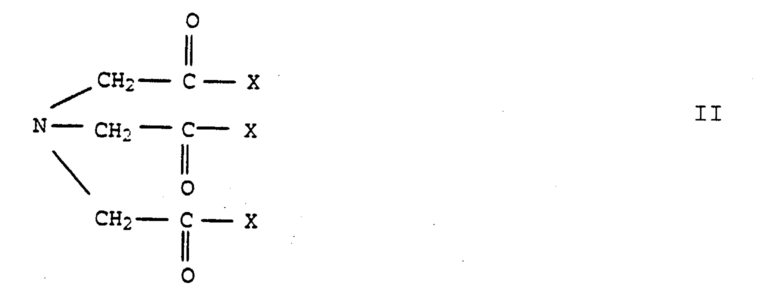 dans lesquelles
A représente un groupement alkyle à chaîne linéaire ou ramifiée avec 2 à 6 atomes de carbone ou un groupement de formule III 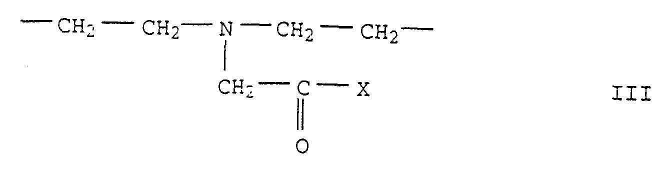
X représente les groupements 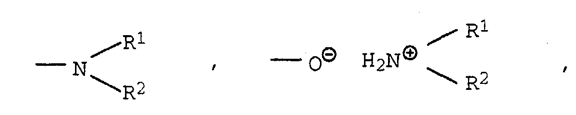
―OH et/ou ―O^{⊖} M^{⊕}
où R¹ et R² sont des groupements aliphatiques à chaîne linéaire avec 10 à 30 atomes de carbone et M est un métal alcalin ou alcalinoterreux,
étant spécifié que les deux conditions suivantes sont remplies, à savoir que X représente
a) au moins un groupement 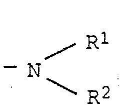 et
b) au moins un groupement -OH- et/ou -O^{⊖}M^{⊕}.

2. Compositions de distillats moyens de pétrole selon la revendication 1, caractérisées en ce qu'elles contiennent des composés de formule I et/ou II, dans lesquelles R¹ et R² représentent un groupement aliphatique à chaîne linéaire avec 14 à 22 atomes de carbone.

3. Compositions de distillats moyens de pétrole selon la revendication 1, caractérisées en ce qu'elles contiennent des composés de formule I, dans laquelle X est mis trois fois pour le groupement 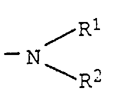 et une fois pour le groupement -OH ou -O^{⊖}M^{⊕}.

4. Compositions de distillats moyens de pétrole selon la revendication 2, caractérisées en ce que R¹ et R² représentent un groupement aliphatique à chaîne linéaire avec 16 à 18 atomes de carbone.
